# EUROPEAN PATENT APPLICATION

(11) **EP 4 592 280 A1**
(43) Date of publication of application: **30.07.2025**
(21) Application number: 23868139.9
(22) Date of filing: 15.09.2023
(51) Int. Cl.: C07D 307/52, C08G 59/50

(54) **AMINO COMPOSITION AND METHOD FOR PRODUCING SAME, EPOXY RESIN CURING AGENT, AND EPOXY RESIN COMPOSITION AND CURED PRODUCT OF SAME**

(30) Priority: 20.09.2022 JP 2022148907
(71) Applicant: MITSUBISHI GAS CHEMICAL COMPANY, INC., Chiyoda-ku Tokyo 100-8324 (JP)
(72) Inventor: OHNO Yuma, Hiratsuka-shi, Kanagawa 254-0016 (JP); AKAI Yuka, Hiratsuka-shi, Kanagawa 254-0016 (JP); KOUNO Kazuki, Hiratsuka-shi, Kanagawa 254-0016 (JP)
(74) Representative: Gille Hrabal Partnerschaftsgesellschaft mbB Patentanwälte
(86) International application number: PCT/JP2023/033656
(87) International publication number: WO 2024/063018

(57) **Abstract**

An amino composition containing an amino compound (A) represented by the following general formula (1) and an amino compound (B) represented by the following general formula (3), wherein a molar ratio between the amino compound (A) and the amino compound (B) [(A)/(B)] is 10/90 to 99/1 and a method for producing the same, an epoxy resin curing agent containing the composition, an epoxy resin composition, and a cured product thereof,

NH₂-CH₂-X-CH₂-NH-R¹ (1)

wherein R¹ is a monovalent group represented by the following general formula (2) and X is a phenylene group, 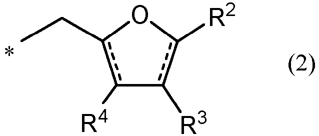 wherein a dashed line indicates presence or absence of a π bond, R² to R⁴ are each independently a hydrogen atom or an alkyl group that has 1 to 4 carbon atoms and optionally has a hydroxy group, and * represents a bonding position,

R¹-NH-CH₂-X-CH₂-NH-R¹ (3)

wherein R¹ and X are the same as above.

## Description

### Technical Field

The present invention relates to an amino composition and a method for producing the same, an epoxy resin curing agent, an epoxy resin composition, and a cured product thereof.

### Background Art

A polyamine compound is known as a kind of epoxy resin curing agent. Epoxy resin compositions using a polyamine compound as an epoxy resin curing agent are used not only in the field of paints such as corrosion-resistant paints for boats and ships, bridges, and onshore and offshore iron structures but also in the field of civil engineering and architecture as lining, reinforcing, and repairing materials for concrete structures, floor materials for architectural structures, linings for water supply and sewerage systems, paving materials, adhesives, and the like.

Among these, for epoxy resin compositions used for paints, it is important to provide coating films having excellent appearance, water resistance, chemical resistance, and physical properties.

Xylylenediamine as a kind of aliphatic polyamine compound can quickly cure an epoxy resin when used as an epoxy resin curing agent and is superior in low-temperature curability, chemical resistance, etc. to other aliphatic polyamines. However, xylylenediamine is likely to absorb carbon dioxide and water vapor in air to generate a carbamic acid salt, and therefore a coating film of an epoxy resin composition using xylylenediamine as an epoxy resin curing agent is likely to blush, which tends to deteriorate the appearance of the coating film.

In order to improve such blushing of a coating film, the use of a modified product of xylylenediamine as an epoxy resin curing agent has been studied.

For example, Patent Literature 1 discloses an epoxy resin curing agent obtained by reacting a predetermined derivative of maleic acid or fumaric acid and a predetermined polyoxyalkyleneamine in a specific ratio and adding a predetermined amount of a polyamine compound such as m-xylylenediamine thereto to further perform a reaction, and states that the curing agent is less likely to be affected by carbon dioxide (surface stickiness of a cured product, blushing).

Patent Literature 2 discloses that a curing agent composition for epoxy resin containing a polyamine compound, which is a reaction product of a compound having at least one glycidyl group in one molecule and a diamine such as xylylenediamine, a predetermined polyether-modified polysiloxane, and a predetermined amino group-modified polysiloxane can solve a problem that appearance is deteriorated due to blushing caused by a reduction in water resistance, which makes it possible to provide an epoxy resin composition excellent in surface appearance such as transparency, drying efficiency (ease of drying), adhesiveness to base materials, and water resistance.

Generally, modified products of a polyamine used as epoxy resin curing agents have high viscosity and are therefore, in most cases, diluted with a non-reactive diluent or the like before use. However, the use of a non-reactive diluent may reduce the chemical resistance etc. of a coating film of an epoxy resin composition containing such a curing agent.

For this reason, as a low-viscosity modified product of xylylenediamine, an amino compound has been studied which is obtained by an addition reaction between a diamine such as xylylenediamine and an alkenyl compound such as styrene (Patent Literature 3).

Patent Literature 4 discloses a latent curing agent to be mixed into an adhesive that is polymerized and cured by an amine used as a curing agent, the latent curing agent being a reaction product of a polyamine having two or more primary amino groups and furfural. However, the reaction product is an imine (aldimine) and is therefore different from an amino compound. Further, Patent Literature 4 states that the latent curing agent is one such that an amine generated by hydrolysis treatment of the aldimine, that is, the polyamine functions as a curing agent. That is, the reaction product, which is a modified product of the polyamine, itself does not function as an epoxy resin curing agent.

Patent Literature 5 discloses a disinfectant for agriculture and gardening, which contains, as its active ingredient, a compound obtained by reducing a compound obtained from 1 mol of xylylenediamine and 2 mol of an aldehyde having a predetermined structure. However, in Patent Literature 5, there is no description about an effect obtained by using the compound as an epoxy resin curing agent.

### Citation List

### Patent Literatures

PTL1: JP 11-35661 A
PTL2: JP 2007-186693 A
PTL3: JP 2002-161076 A
PTL4: JP 10-139849 A
PTL5: JP 47-23530 A

### Summary of Invention

### Technical Problem

The amino compound disclosed as a modified product of xylylenediamine in Patent Literature 3 has low viscosity and excellent water resistance, but there is room for improvement in the resistance to water-containing methanol of a coating film of an epoxy resin composition obtained using the amino compound as an epoxy resin curing agent.

It is an object of the present invention to provide an amino composition that has low viscosity and can be used as an epoxy resin curing agent to form a coating film having excellent resistance to water-containing methanol and a method for producing the same, an epoxy resin curing agent containing the composition, an epoxy resin composition, and a cured product thereof.

### Solution to Problem

The present inventors have found that the above object can be achieved by an amino composition containing a modified product of a diamine having a predetermined structure in a predetermined ratio.

Specifically, the present invention relates to the following aspects.
[1] An amino composition containing an amino compound (A) represented by the following general formula (1) and an amino compound (B) represented by the following general formula (3), wherein a molar ratio between the amino compound (A) and the amino compound (B) [(A)/(B)] in the composition is 10/90 to 99/1,

   NH₂-CH₂-X-CH₂-NH-R¹ (1)

   wherein R¹ is a monovalent group represented by the following general formula (2) and X is a phenylene group, 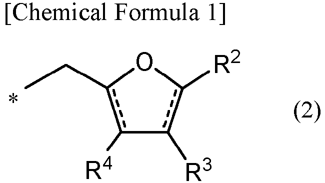
   wherein a dashed line indicates presence or absence of a *π* bond, R² to R⁴ are each independently a hydrogen atom or an alkyl group that has 1 to 4 carbon atoms and optionally has a hydroxy group, and * represents a bonding position,

      R¹-NH-CH₂-X-CH₂-NH-R¹ (3)
   wherein R¹ and X are the same as above.
[2] An epoxy resin curing agent containing the amino composition according to the above [1].
[3] An epoxy resin composition containing an epoxy resin and the epoxy resin curing agent according to the above [2].
[4] A cured product of the epoxy resin composition according to the above [3].
[5] A method for producing the amino composition according to the above [1], the method including the following step (1) and step (2) in this order:
   step (1): the step of reacting a diamine represented by the following general formula (4) and an aldehyde compound represented by the following general formula (5) to obtain an imine; and
   step (2): the step of reducing the imine obtained in the step (1),

      NH₂-CH₂-X- CH₂-NH₂ (4)

      wherein X is the same as above, 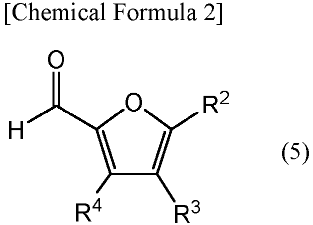
      wherein R² to R⁴ are the same as above.
[6] The method for producing the amino composition according to the above [5], wherein in the step (1), 0.8 to 1.8 mol of the aldehyde compound represented by the above general formula (5) is reacted per 1 mol of the diamine represented by the above general formula (4).

### Advantageous Effects of Invention

The present invention makes it possible to provide an amino composition that has low viscosity and can be used as an epoxy resin curing agent to form a coating film having excellent resistance to water-containing methanol and a method for producing the same, an epoxy resin curing agent containing the composition, an epoxy resin composition, and a cured product thereof.

### Description of Embodiments

### [Amino composition]

An amino composition of the present invention is an amino composition containing an amino compound (A) represented by the following general formula (1) and an amino compound (B) represented by the following general formula (3), wherein a molar ratio between the amino compound (A) and the amino compound (B) [(A)/(B)] in the composition is 10/90 to 99/1,

NH₂-CH₂-X-CH₂-NH-R¹ (1)

wherein R¹ is a monovalent group represented by the following general formula (2) and X is a phenylene group, 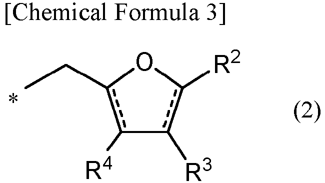
wherein a dashed line indicates the presence or absence of a π bond, R² to R⁴ are each independently a hydrogen atom or an alkyl group that has 1 to 4 carbon atoms and optionally has a hydroxy group, and * represents a bonding position, R¹-NH-CH₂-X-CH₂-NH-R¹ (3)
wherein R¹ and X are the same as above.

An epoxy resin curing agent containing the amino composition of the present invention has low viscosity, and further, the use of an epoxy resin composition containing the epoxy resin curing agent makes it possible to form a coating film having excellent resistance to water-containing methanol.

The reason why such effects are obtained by the amino composition of the present invention is not clear but can be considered as follows.

The amino composition of the present invention is obtained by, for example, reducing a reaction product (imine) of xylylenediamine and furfural or a derivative thereof, and therefore from such a viewpoint, the amino composition of the present invention can be said as a kind of modified product of xylylenediamine. It is considered that, in the above production method, the use of xylylenediamine as a reaction starting material of the amino composition and, in addition, introduction of an aromatic substituent, such as a furan ring, as a substituent for an amino group in the xylylenediamine improve hydrophobicity so that the water resistance of a coating film improves. Further, when xylylenediamine is modified with an epoxy compound or the like, a resulting modified product generally has high viscosity, but it is considered that an increase in viscosity can be prevented by modifying xylylenediamine with furfural or a derivative thereof. Further, the amino composition of the present invention does not need to reduce its viscosity by adding a non-reactive diluent or the like, and therefore it is considered that a coating film of an epoxy resin composition using the amino composition as an epoxy resin curing agent can be prevented from being reduced in resistance to water-containing methanol due to addition of a non-reactive diluent.

The amino composition of the present invention contains the amino compound (A) represented by the above general formula (1) and the amino compound (B) represented by the above general formula (3) in a predetermined ratio. The amino compound (A) is a compound obtained by reducing an imine as a reaction product obtained by reacting the diamine and aldehyde compound in a molar ratio of 1/1 (hereinafter, the compound will be sometimes referred to as a "monoadduct"), and the amino compound (B) is a compound obtained by reducing an imine as a reaction product obtained by reacting the diamine and aldehyde compound in a molar ratio of 1/2 (hereinafter, the compound will be sometimes referred to as a "diadduct").

Patent Literature 5 discloses the amino compound (B) as a compound obtained by reducing a reaction product obtained by reacting xylylenediamine and furfural in a molar ratio of 1/2. However, when the amino composition of the present invention containing the amino compound (A) being a monoadduct in a high ratio is used as an epoxy resin curing agent, a coating film of an epoxy resin composition obtained using the epoxy resin curing agent is superior in methanol resistance as compared to when the amino compound (B) being a diadduct is used as an epoxy resin curing agent.

### <Amino compound (A)>

The amino composition of the present invention contains an amino compound (A) represented by the following general formula (1): NH₂-CH₂-X-CH₂-NH-R¹ (1)
wherein R¹ is a monovalent group represented by the following general formula (2) and X is a phenylene group, 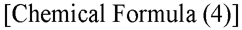 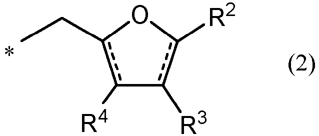
wherein a dashed line indicates the presence or absence of a π bond, R² to R⁴ are each independently a hydrogen atom or an alkyl group that has 1 to 4 carbon atoms and optionally has a hydroxy group, and * represents a bonding position.

In the above general formula (1), X is a phenylene group and is any one of a 1,2-phenylene group, a 1,3-phenylene group, and a 1,4-phenylene group. From the viewpoint of low viscosity, fast curing at the time when the amino composition of the present invention is used as an epoxy resin curing agent, and improvement in hardness, appearance, water resistance, and resistance to water-containing methanol of a coating film of an epoxy resin composition obtained using the epoxy resin curing agent, X is preferably a 1,3-phenylene group or a 1,4-phenylene group, more preferably a 1,3-phenylene group.

In the above general formula (2), a dashed line indicates the presence or absence of a π bond. The π bonds preferably present on the dashed lines but a part of the π bonds may be reduced to be absent

In the above general formula (2), the alkyl group represented by each of R² to R⁴ is preferably an alkyl group having 1 to 3 carbon atoms, more preferably a methyl group or an ethyl group, even more preferably a methyl group.

From the viewpoint of low viscosity, fast curing at the time when the amino composition of the present invention is used as an epoxy resin curing agent, and improvement in hardness, appearance, water resistance, and resistance to water-containing methanol of a coating film of an epoxy resin composition obtained using the epoxy resin curing agent, R² to R⁴ are preferably each a hydrogen atom, a methyl group, or a hydroxymethyl group, more preferably each a hydrogen atom or a methyl group, and even more preferably, R² to R⁴ are all hydrogen atoms or R² is a methyl group and R³ and R⁴ are hydrogen atoms, and still even more preferably, R² to R⁴ are all hydrogen atoms.

### <Amino compound (B)>

The amino composition of the present invention contains an amino compound (B) represented by the following general formula (3):

R¹-NH-CH₂-X-CH₂-NH-R¹ (3)

wherein R¹ and X are the same as above.

In the above general formula (3), R¹ is a monovalent group represented by the above general formula (2), and preferred examples thereof are also the same as above.

In the above general formula (3), X is a phenylene group and is any one of a 1,2-phenylene group, a 1,3-phenylene group, and a 1,4-phenylene group. From the viewpoint of low viscosity, fast curing at the time when the amino composition of the present invention is used as an epoxy resin curing agent, and improvement in hardness, appearance, water resistance, and resistance to water-containing methanol of a coating film of an epoxy resin composition obtained using the epoxy resin curing agent, X is preferably a 1,3-phenylene group or a 1,4-phenylene group, more preferably a 1,3-phenylene group.

The molar ratio between the amino compound (A) and the amino compound (B) [(A)/(B)] in the amino composition is, from the viewpoint of low viscosity, fast curing at the time when the amino composition is used as an epoxy resin curing agent, and improvement in hardness, appearance, water resistance, and resistance to water-containing methanol of a coating film of an epoxy resin composition obtained using the epoxy resin curing agent, 10/90 to 99/1, preferably 20/80 to 99/1, more preferably 30/70 to 99/1, still even preferably 40/60 to 99/1, still even more preferably 50/50 to 99/1.

The above molar ratio can specifically be determined by a method that will be described in Examples.

The amino composition may contain, in addition to the amino compound (A) and the amino compound (B), a by-product and a diamine (xylylenediamine), which is represented by a general formula (4) and will be described later, as an unreacted raw material. However, from the viewpoint of low viscosity, fast curing at the time when the amino composition is used as an epoxy resin curing agent, and improvement in hardness, appearance, water resistance, and resistance to water-containing methanol of a coating film of an epoxy resin composition obtained using the epoxy resin curing agent, the total content of the amino compound (A) and the amino compound (B) in the amino composition is preferably 70 mass% or more, more preferably 80 mass% or more, even more preferably 90 mass% or more, still even more preferably 95 mass% or more, still even more preferably 98 mass% or more and is 100 mass% or less.

The content of the xylylenediamine, which is an unreacted raw material, in the amino composition is, from the viewpoint of improvement in hardness, appearance, water resistance, and resistance to water-containing methanol of a coating film of an epoxy resin composition obtained using the amino composition as an epoxy resin curing agent, preferably 5 mass% or less, more preferably 2 mass% or less, even more preferably 1 mass% or less.

The total content of the amino compound (A) and the amino compound (B) and the content of xylylenediamine, which is an unreacted raw material, in the amino composition can be measured by gas chromatographic (GC) analysis.

The viscosity of the amino composition at 25°C is, from the viewpoint of obtaining an epoxy resin curing agent having low viscosity, preferably 500 mPa·s or less, more preferably 300 mPa·s or less, even more preferably 200 mPa·s or less, still even more preferably 100 mPa·s or less, still even more preferably 90 mPa·s or less and is usually 10 mPa·s or more.

The viscosity of the amino composition at 25°C can specifically be measured using a type-E viscometer by a method that will be described in Examples.

### [Method for producing amino composition]

A method for producing the amino composition of the present invention (hereinafter referred to also as a "production method of the present invention") preferably includes the following step (1) and step (2) in this order:
step (1): the step of reacting a diamine represented by the following general formula (4) and an aldehyde compound represented by the following general formula (5) to obtain an imine; and
step (2): the step of reducing the imine obtained in the step (1),

   NH₂-CH₂-X- CH₂-NH₂ (4)

   wherein X is the same as above, 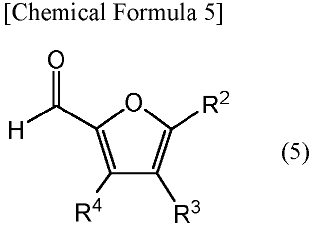
   wherein R² to R⁴ are the same as above.

### <Step (1)>

In the step (1), a diamine represented by the above general formula (4) and an aldehyde compound represented by the above general formula (5) are reacted to obtain an imine.

In the above general formula (4), X is a phenylene group and is any one of a 1,2-phenylene group, a 1,3-phenylene group, and a 1,4-phenylene group. From the viewpoint of low viscosity, fast curing at the time when a resulting amino composition is used as an epoxy resin curing agent, and improvement in hardness, appearance, water resistance, and resistance to water-containing methanol of a coating film of an epoxy resin composition obtained using the epoxy resin curing agent, X is preferably a 1,3-phenylene group or a 1,4-phenylene group, more preferably a 1,3-phenylene group.

A specific example of the diamine represented by the above general formula (4) is at least one selected from the group consisting of o-xylylenediamine, m-xylylenediamine, and p-xylylenediamine. From the viewpoint of low viscosity, fast curing at the time when a resulting amino composition is used as an epoxy resin curing agent, and improvement in hardness, appearance, water resistance, and resistance to water-containing methanol of a coating film of an epoxy resin composition obtained using the epoxy resin curing agent, the diamine represented by the above general formula (4) is preferably at least one selected from the group consisting of m-xylylenediamine and p-xylylenediamine, more preferably m-xylylenediamine or a mixture of m-xylylenediamine and p-xylylenediamine, even more preferably m-xylylenediamine.

R² to R⁴ in the above general formula (5) are each independently a hydrogen atom or an alkyl group that has 1 to 4 carbon atoms and optionally has a hydroxy group. The alkyl group represented by each of R² to R⁴ is preferably an alkyl group having 1 to 3 carbon atoms, more preferably a methyl group or an ethyl group, even more preferably a methyl group.

From the viewpoint of low viscosity of resulting amino compound and amino composition, fast curing at the time when the amino compound or the amino composition is used as an epoxy resin curing agent, and improvement in hardness, appearance, water resistance, and resistance to water-containing methanol of a coating film of an epoxy resin composition obtained using the epoxy resin curing agent, R² to R⁴ are preferably each a hydrogen atom, a methyl group, or a hydroxymethyl group, more preferably each a hydrogen atom or a methyl group, and even more preferably, R² to R⁴ are all hydrogen atoms or R² is a methyl group and R³ and R⁴ are hydrogen atoms, and still even more preferably R² to R⁴ are all hydrogen atoms.

Specific examples of the aldehyde compound represented by the above general formula (5) include furfural, 3-methyl furfural, 4-methyl furfural, 5-methyl furfural, 3-hydroxymethyl furfural, 4-hydroxymethyl furfural, 5-hydroxymethyl furfural, 4,5-dimethyl furfural, 3-ethyl furfural, 4-ethyl furfural, 5-ethyl furfural, 3-propyl furfural, 4-propyl furfural, 5-propyl furfural, 3-butyl furfural, 4-butyl furfural, and 5-butyl furfural, and any one or two or more of them may be used.

Among these, from the viewpoint of low viscosity of resulting amino compound and amino composition, fast curing at the time when the amino compound or the amino composition is used as an epoxy resin curing agent, and improvement in hardness, appearance, water resistance, and resistance to water-containing methanol of a coating film of an epoxy resin composition obtained using the epoxy resin curing agent, the aldehyde compound represented by the above general formula (5) is preferably at least one selected from the group consisting of furfural, 3-methyl furfural, 4-methyl furfural, 5-methyl furfural, 3-hydroxymethyl furfural, 4-hydroxymethyl furfural, 5-hydroxymethyl furfural, and 4,5-dimethyl furfural, more preferably at least one selected from the group consisting of furfural and 5-methyl furfural, even more preferably furfural.

In the step (1), from the viewpoint of obtaining an amino compound represented by the above general formula (1) and an amino composition containing the amino compound in high yield, 0.8 to 1.8 mol, preferably 1.0 to 1.8 mol, more preferably 1.0 to 1.5 mol, still even preferably 1.0 to 1.3 mol of the aldehyde compound represented by the above general formula (5) is reacted per 1 mol of the diamine represented by the above general formula (4).

The reaction between the diamine represented by the above general formula (4) and the aldehyde compound represented by the above general formula (5) is preferably performed under heating and stirring conditions. From the viewpoint of improving reaction efficiency and the viewpoint of preventing thermal deterioration of the diamine and the aldehyde compound as raw materials, a reaction temperature is preferably 40 to 120°C, more preferably 50 to 95°C. A reaction time may appropriately be selected, but is usually 15 minutes to 6 hours.

The reaction can be performed in a reaction solvent, but may be performed in the absence of solvent.

By performing the above reaction in the step (1), it is possible to obtain an imine as a reaction product of the diamine represented by the above general formula (4) and the aldehyde compound represented by the above general formula (5). The reaction product obtained in the step (1) may be purified or directly subjected to the step (2) without being purified.

### <Step (2)>

In the step (2), the imine obtained in the step (1) is reduced to convert it to an amine. Reduction of the imine is preferably performed by hydrogen addition (hydrogenation) under heating and pressurized conditions in the presence of a catalyst.

The catalyst used in the step (2) may be a publicly-known hydrogenation catalyst, and examples thereof include: a supported heterogeneous hydrogenation catalyst in which a metal such as Ni, Pt, Pd, or Ru is supported by carbon, silica, alumina, diatomaceous earth, or the like; a so-called Ziegler-type hydrogenation catalyst using a transition metal salt such as an organic acid salt or acetylacetone salt of Ni, Co, Fe, Cr, or the like and a reducing agent such as organoaluminum; and a homogeneous hydrogenation catalyst such as a so-called organometallic complex such as an organometallic compound of Ti, Ru, Rh, Zr, or the like.

From the viewpoint of improving reaction efficiency and the viewpoint of preventing a side reaction, a temperature during the hydrogenation reaction is preferably 0°C or higher, more preferably 10°C or higher, even more preferably 20°C or higher and is preferably 200°C or lower, more preferably 150°C or lower, even more preferably 100°C or lower.

From the viewpoint of improving reaction efficiency and the viewpoint of preventing a side reaction, a pressure during the hydrogenation reaction is preferably 0.01 MPaG or more, more preferably 0.1 MPaG or more, even more preferably 0.3 MPaG or more and is preferably 10 MPaG or less, more preferably 3 MPaG or less.

A reaction time is not limited, but is preferably 3 minutes or more, more preferably 10 minutes or more, even more preferably 30 minutes or more and is preferably 24 hours or less, more preferably 12 hours or less, even more preferably 8 hours or less.

The hydrogenation reaction may be performed in the presence of a solvent. The solvent is not limited as long as it does not inhibit the hydrogenation reaction, and examples of such a solvent include hydrocarbon solvents such as aliphatic hydrocarbons such as pentane, hexane, iso-pentane, heptane, octane, and iso-octane, alicyclic hydrocarbons such as cyclopentane, methylcyclopentane, cyclohexane, methylcyclohexane, and ethylcyclohexane, and aromatic hydrocarbons such as toluene, ethyl benzene, and xylene. These may be used singly or in combination of two or more of them.

After the hydrogenation reaction, the catalyst is removed from an obtained reaction liquid and, if necessary, the reaction liquid is purified by distillation. In this way, the amino compound of the present invention or the amino composition can be obtained.

It should be noted that the method for producing the amino composition is not limited to the above method. The amino composition can be produced also by, for example, a method in which a diamine represented by the above general formula (4) and an amine represented by the following general formula (6) are subjected to a deammoniation reaction in the presence of a catalyst; a method in which a diamine represented by the above general formula (4) and an alcohol represented by the following general formula (7) are reacted in a hydrogen atmosphere in the presence of a catalyst; or a method in which a diamine represented by the above general formula (4) and a chloride represented by the following general formula (8) are reacted. At this time, the amount of each of the compounds represented by the general formulas (6) to (8) reacted per mol of the diamine represented by the above general formula (4) is preferably 0.8 to 1.8 mol, more preferably 1.0 to 1.8 mol, even more preferably 1.0 to 1.5 mol, still even more preferably 1.0 to 1.3 mol. 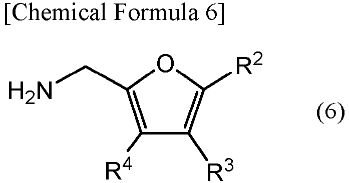 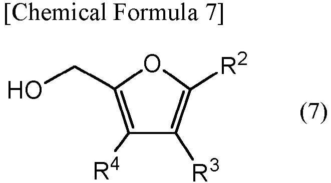 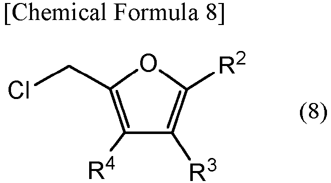 wherein R² to R⁴ are the same as above.

### [Epoxy resin curing agent]

An epoxy resin curing agent of the present invention contains the amino composition of the present invention. The epoxy resin curing agent has low viscosity and can be used for an epoxy resin composition to form a coating film having excellent resistance to water-containing methanol.

From the viewpoint of low viscosity, fast curing, and improvement in hardness, appearance, water resistance, and resistance to water-containing methanol of a coating film of an epoxy resin composition obtained using the epoxy resin curing agent, the content of the above-described amino composition in the epoxy resin curing agent is preferably 30 mass% or more, more preferably 50 mass% or more, even more preferably 60 mass% or more, still even more preferably 70 mass% or more, still even more preferably 80 mass% or more, still even more preferably 90 mass% or more and is 100 mass% or less.

The epoxy resin curing agent of the present invention may contain another curing agent component in addition to the above-described amino composition. The curing agent component herein means a component that is contained in the epoxy resin curing agent and has two or more active hydrogen atoms that may react with epoxy groups in an epoxy resin.

Examples of the another curing agent component include an amine-based curing agent, a phenol-based curing agent, and an acid anhydride-based curing agent. From the viewpoint of fast curing, an amine-based curing agent is preferred.

Examples of the amine-based curing agent include a polyamine compound other than the amino composition and a modified product thereof. The polyamine compound is not limited as long as it is a compound having at least two amino groups in its molecule.

Examples of the polyamine compound or the modified product thereof include: chain-like aliphatic polyamine compounds such as ethylenediamine, diethylenetriamine, triethylenetetramine, tetraethylenepentamine, pentaethylenehexamine, hexamethylenediamine, 2-methylpentamethylenediamine, and trimethylhexamethylenediamine; polyamine compounds having an alicyclic structure, such as 1,2-bis(aminomethyl)cyclohexane, 1,3-bis(aminomethyl)cyclohexane, 1,4-bis(aminomethyl)cyclohexane, menthenediamine, isophoronediamine, norbornanediamine, tricyclodecanediamine, adamantanediamine, diaminocyclohexane, 1,4-diamino-2-methylcyclohexane, 1,4-diamino-3,6-diethylcyclohexane, diaminodiethylmethylcyclohexane, 3,3'-dimethyl-4,4'-diaminodicyclohexylmethane, and 4,4'-diaminodicyclohexylmethane; polyamine compounds having an aromatic ring, such as o-xylylenediamine, m-xylylenediamine, p-xylylenediamine, phenylenediamine, diaminodiphenylmethane, and diaminodiphenylsulfone; polyamine compounds having a heterocyclic structure, such as N-aminomethylpiperazine and N-aminoethylpiperazine; polyether polyamine compounds; a reaction product of the above-mentioned polyamine compound and an epoxy compound having at least one epoxy group, an unsaturated hydrocarbon compound, a carboxylic acid, or a derivative thereof; a Mannich reaction product obtained by reacting the above-mentioned polyamine compound, a phenolic compound, and an aldehyde compound; and ketoimines (ketimines) obtained by reacting the above-mentioned polyamine compound and a ketone compound. These may be used singly or in combination of two or more of them.

When the another curing agent component is used, the content of the another curing agent component in the epoxy resin curing agent is preferably 1 mass% or more, more preferably 5 mass% or more. The upper limit of the content is not limited as long as the effects of the present invention are not impaired, but is preferably 70 mass% or less, more preferably 50 mass% or less, even more preferably 40 mass% or less, still even more preferably 30 mass% or less, still even more preferably 20 mass% or less.

The epoxy resin curing agent of the present invention may further contain a publicly-known curing accelerator, a non-reactive diluent, and others.

Examples of the curing accelerator include phenolic compounds, organic acids, organic acid salts, tertiary amines, quaternary ammonium salts, imidazoles, organophosphorus-based compounds, quaternary phosphonium salts, diazabicycloalkenes, organometallic salt compounds, boron compounds, and metallic halides.

Examples of the non-reactive diluent include benzyl alcohol, furfuryl alcohol, tetrafurfuryl alcohol, and aromatic hydrocarbon formaldehyde resins, and any one or two or more of them may be used.

However, from the viewpoint of improving the resistance to water-containing methanol of a coating film of an epoxy resin composition obtained using the epoxy resin curing agent of the present invention, the content of the non-reactive diluent in the epoxy resin curing agent of the present invention is preferably low. The content of the non-reactive diluent in the epoxy resin curing agent is preferably 20 mass% or less, more preferably 10 mass% or less, even more preferably 5 mass% or less, still even more preferably 1 mass% or less, still even more preferably 0 mass%.

The active hydrogen equivalent weight of the epoxy resin curing agent is, from the viewpoint of low viscosity, fast curing, and improvement in hardness, appearance, water resistance, and resistance to water-containing methanol of a coating film of an epoxy resin composition obtained using the epoxy resin curing agent, preferably 80 or more and is, from the viewpoint of improving curability, preferably 150 or less, more preferably 120 or less. The active hydrogen equivalent weight (hereinafter referred to also as "AHEW") herein refers to the mass of the epoxy resin curing agent per mol of amino group-derived active hydrogen. The AHEW of the curing agent can be calculated from an amine value.

### [Epoxy resin composition]

An epoxy resin composition of the present invention contains an epoxy resin and the above-described epoxy resin curing agent. The epoxy resin composition has a high curing rate, and a resulting coating film has excellent hardness, water resistance, and resistance to water-containing methanol.

### <Epoxy resin>

The epoxy resin as a main agent of the epoxy resin composition may be any one of a saturated or unsaturated aliphatic compound or alicyclic compound, an aromatic compound, and a heterocyclic compound. From the viewpoint of improving a curing rate and the viewpoint of forming a coating film having excellent hardness, appearance, water resistance, and resistance to water-containing methanol, an epoxy resin having an aromatic ring or an alicyclic structure in its molecule is preferred.

A specific example of the epoxy resin is at least one resin selected from the group consisting of an epoxy resin having a glycidylamino group derived from m-xylylenediamine, an epoxy resin having a glycidylamino group derived from p-xylylenediamine, an epoxy resin having a glycidylamino group derived from 1,3-bis(aminomethyl)cyclohexane, an epoxy resin having a glycidylamino group derived from 1,4-bis(aminomethyl)cyclohexane, an epoxy resin having a glycidylamino group derived from diaminodiphenylmethane, an epoxy resin having a glycidylamino group and/or a glycidyloxy group derived from p-aminophenol, an epoxy resin having a glycidyloxy group derived from bisphenol A, an epoxy resin having a glycidyloxy group derived from bisphenol F, an epoxy resin having a glycidyloxy group derived from phenol novolac, and an epoxy resin having a glycidyloxy group derived from resorcinol. The above-mentioned epoxy resins may be used in combination of two or more of them.

Among these, from the viewpoint of obtaining a curing rate and the viewpoint of forming a coating film having excellent hardness, water resistance, and resistance to water-containing methanol preferred is an epoxy resin containing, as its main component, at least one selected from the group consisting of an epoxy resin having a glycidylamino group derived from m-xylylenediamine, an epoxy resin having a glycidylamino group derived from p-xylylenediamine, an epoxy resin having a glycidyloxy group derived from bisphenol A, and an epoxy resin having a glycidyloxy group derived from bisphenol F, and from the viewpoint of improving a curing rate, the viewpoint of forming a coating film having excellent hardness, appearance, water resistance, and resistance to water-containing methanol and the viewpoint of availability and economy, more preferred is an epoxy resin containing, as its main component, an epoxy resin having a glycidyloxy group derived from bisphenol A.

It should be noted that the term "main component" here means that another component may be contained without departing from the spirit of the present invention, and means that the content thereof is preferably 50 to 100 mass%, more preferably 70 to 100 mass%, even more preferably 90 to 100 mass% of the total mass.

From the viewpoint of improving handleability, the epoxy resin as a main agent may contain a reactive diluent other than the above-described epoxy resin. The reactive diluent may be a low-molecular-weight compound having at least one epoxy group, and examples thereof include: aromatic monoglycidyl ethers such as phenylglycidyl ether and cresylglycidyl ether; alkylmonoglycidyl ethers such as butylglycidyl ether, hexylglycidyl ether, octylglycidyl ether, decylglycidyl ether, laurylglycidyl ether, and tetradecylglycidyl ether; and diglycidyl ethers of aliphatic diols, such as 1,3-propanedioldiglycidyl ether, 1,4-butanedioldiglycidyl ether, neopentylglycoldiglycidyl ether, and 1,6-hexanedioldiglycidyl ether.

The above-mentioned reactive diluents may be used singly or in combination of two or more of them.

The content ratio between the epoxy resin and the epoxy resin curing agent in the epoxy resin composition of the present invention is set in such a manner that the ratio of the number of amino group-derived active hydrogen atoms in the epoxy resin curing agent to the number of epoxy groups in the epoxy resin (the number of amino group-derived active hydrogen atoms in the epoxy resin curing agent/the number of epoxy groups in the epoxy resin) is preferably 1/0.5 to 1/2, more preferably 1/0.75 to 1/1.5, even more preferably 1/0.8 to 1/1.2.

The epoxy resin content and the epoxy resin curing agent content in the epoxy resin composition are not limited as long as the ratio of the number of amino group-derived active hydrogen atoms in the epoxy resin curing agent to the number of epoxy groups in the epoxy resin is preferably within the above range. However, from the viewpoint of improving a curing rate and the viewpoint of forming a coating film having excellent hardness, appearance, water resistance, and resistance to water-containing methanol, the epoxy resin content and the epoxy resin curing agent content in the epoxy resin composition are preferably within the following ranges, respectively.

The epoxy resin content in the epoxy resin composition is preferably 40 mass% or more, more preferably 50 mass% or more, even more preferably 60 mass% or more and is preferably 80 mass% or less, more preferably 75 mass% or less.

The epoxy resin curing agent content in the epoxy resin composition is preferably 20 mass% or more, more preferably 25 mass% or more and is preferably 60 mass% or less, more preferably 50 mass% or less, even more preferably 40 mass% or less.

Further, the epoxy resin curing agent content in the epoxy resin composition is preferably 20 to 60 parts by mass, more preferably 30 to 60 parts by mass, still even preferably 40 to 60 parts by mass per 100 parts by mass of the epoxy resin as a main agent.

From the viewpoint of low viscosity, fast curing, and improvement in hardness, appearance, water resistance, and resistance to water-containing methanol of a resulting coating film, the total content of the epoxy resin and the epoxy resin curing agent in the epoxy resin composition is preferably 40 mass% or more, more preferably 50 mass% or more, even more preferably 60 mass% or more, still even more preferably 70 mass% or more, still even more preferably 80 mass% or more, still even more preferably 90 mass% or more and is 100 mass% or less.

The epoxy resin composition of the present invention may further contain a modifying component such as a filler or a plasticizer, a flow modifying component such as a thixotropic agent, and another component such as a pigment, a leveling agent, a tackifier, or elastomer fine particles depending on its intended use.

The epoxy resin composition of the present invention may contain the above-described non-reactive diluent and a solvent (water and a volatile solvent) other than the non-reactive diluent, but the content thereof in the epoxy resin composition is preferably 5 mass% or less, more preferably 2 mass% or less, even more preferably 1 mass% or less.

### <Method for producing epoxy resin composition>

A method for producing the epoxy resin composition of the present invention is not limited, and the epoxy resin composition of the present invention can be produced by mixing the epoxy resin, the epoxy resin curing agent, and another optional component by a publicly-known method using a publicly-known device. The order of mixing the components of the epoxy resin composition is not limited, either. The epoxy resin curing agent may be prepared first and then mixed with the epoxy resin, or the components of the epoxy resin curing agent, another component, and the epoxy resin may be mixed at the same time.

### [Cured product]

A cured product of the epoxy resin composition of the present invention (hereinafter simply referred to also as a "cured product of the present invention") is obtained by curing the above-described epoxy resin composition by a publicly-known method. The curing conditions of the epoxy resin composition are not limited and are appropriately selected depending on the intended use and form of a resulting cured product.

The form of the cured product of the present invention is not limited, either and may appropriately be selected depending on the intended use of the cured product. From the viewpoint that a coating film having excellent hardness, appearance, water resistance, and resistance to water-containing methanol can be formed, the cured product of the epoxy resin composition is preferably in the form of a film.

### <Intended use>

The epoxy resin composition of the present invention is capable of forming a coating film having excellent hardness, appearance, water resistance, and resistance to water-containing methanol and is therefore suitably used for, for example, paints such as marine paints, heavy-duty corrosion-resistant paints, paints for tanks, pipe interior coating paints, exterior paints, and floor paints.

### Examples

The present invention will be described below in detail with reference to examples and comparative examples, but the present invention is not limited to the following examples. It should be noted that amino compositions were analyzed and evaluated by the following methods.

### <Gas chromatographic (GC) analysis>

The content ratio between components in an amino composition was measured by GC analysis. Measurement conditions are as follows.
Device: "7890B GC" manufactured by Agilent Technologies, Inc.
Column: "CP-Sil 8 CB for Amines" manufactured by Agilent Technologies, Inc. (length 30 m, film thickness 0.25 µm, inner diameter 0.25 mm)
Column temperature: 40°C 10 min → heating up at 20°C/min → 250°C 10 min → heating up at 20°C/min → 300°C 10 min
Carrier gas: helium
Carrier gas flow rate: 2.2553 mL/min
Inlet pressure: 22.474 psi (constant pressure mode)
Detector: FID
Inlet temperature: 250°C
Detector temperature: 310°C

### <¹H-NMR analysis>

The structural identification of each component contained in an obtained amino composition was performed by ¹H-NMR analysis. Measurement conditions are as follows.
Nuclear magnetic resonance spectrometer: AVANCEIII-500 manufactured by Bruker BioSpin GmbH.
Probe: 5 mmϕ double resonance, multinuclear probe (BBFO Plus Smart Probe)
Deuterated solvent: deuterated chloroform
Nuclide to be measured: 1H
Measurement temperature: room temperature

### <Compositional analysis of amino composition (molar ratio between amino compound (A) and amino compound (B))>

The molar ratio between an amino compound (A) and an amino compound (B) in an amino composition was calculated from the result of determination of integrated value of protons at benzyl positions of each of the amino compounds by ¹H-NMR analysis performed under the above-described conditions.

### <Measurement of viscosity>

The viscosity of an amino composition (curing agent) at 25°C was measured using a type-E viscometer "TVE-35H viscometer cone plate type" manufactured by Toki Sangyo Co., Ltd.

### <Measurement of active hydrogen equivalent weight (AHEW)>

The AHEW of an amino composition (curing agent) was calculated from the results of determination of a total amine value and a secondary/tertiary amine value with the use of an automatic potentiometric titrator "AT-710S" manufactured by Kyoto Electronics Manufacturing Co., Ltd. The total amine value was measured using a 0.1 mol/L perchloric/acetic acid solution (manufactured by KANTO CHEMICAL CO., INC.), and the secondary/tertiary amine value was measured using 0.1 mol/L hydrochloric acid (2-propanol).

### <Set to touch>

As a base material, a zinc phosphate-treated steel plate (manufactured by PALTEK CORPORATION; SPCC-SDPB-N144 0.8 × 70 × 150 mm) was used. An epoxy resin composition of each example was applied onto the base material using an applicator under conditions of 23°C and 50% R.H. to form a coating film (film thickness just after application: 200 µm). This coating film was stored under conditions of 23°C and 50% R.H. and evaluated by finger touch after a lapse of 1 day and 7 days according to the following criteria. The result is shown in Table 2.
Ex: Excellent (Even when the coating film is pressed with a thumb at a force of about 50 N, there is no stickiness and no fingerprint is left.)
G: Good (Even when the coating film is pressed with a thumb at a force of about 50 N, there is no stickiness, but a fingerprint is left after finger touch.)
F: Fair (When the coating film is pressed with a thumb at a force of about 50 N, there is stickiness).
P: Poor (Uncured)

### <Pencil hardness>

In the same manner as described above, an epoxy resin composition was applied onto a base material (zinc phosphate-treated steel plate) to form a coating film (thickness just after application: 200 µm). This coating film was stored under conditions of 23°C and 50% R.H., and after a lapse of 1 day and 7 days, the pencil hardness of the coating film was measured in accordance with JIS K5600-5-4: 1999. The result is shown in Table 2.

### <Water resistance spot test>

In the same manner as described above, an epoxy resin composition was applied onto a base material (zinc phosphate-treated steel plate) to form a coating film (thickness just after application: 200 µm). This coating film was stored under conditions of 23°C and 50% R.H. After a lapse of 1 day and 7 days, 2 to 3 drops of pure water were dropped with a dropper onto the surface of the coating film, and this spot was covered with a 50-mL screw tube bottle. After a lapse of 24 hours, water was wiped off, and the appearance of the spot was visually observed and evaluated according to the following criteria. The result is shown in Table 2.
Ex: There is no change at all.
G: There is a slight change, but there is no problem for use.
F: Blushing is slightly observed.
P: Significant blushing is observed.

### <Appearance (transparency/gloss)

In the same manner as described above, an epoxy resin composition of each example was applied onto a base material (zinc phosphate-treated steel plate) using an applicator to form a coating film (film thickness just after application: 200 µm). This coating film was stored under conditions of 23°C and 50% R.H., and after a lapse of 1 day, the appearance of the coating film was visually observed to evaluate transparency and gloss according to the following criteria. The results are shown in Table 2.

### (Transparency)

Ex: There is no turbidity.
G: Turbidity is slightly observed, but there is no problem for use.
F: White turbidity is slightly observed.
P: White turbidity is observed.

### (Gloss)

Ex: There is gloss.
G: Gloss is slightly poor, but there is no problem for use.
F: Gloss is poor.
P: There is no gloss.

### <RCI drying time (dust free)>

An epoxy resin composition of each example was applied onto a glass plate (manufactured by Taiyu Kizai K.K., 25 × 348 × 2.0 mm) using a 76-µm gap applicator under conditions of 23°C and 50% R.H. to form a coating film. The glass plate on which the coating film was formed was set on a paint drying time recorder (manufactured by Taiyu Kizai K.K.), and a striation formed by scratching the surface of the coating film with a needle of the recorder was observed to measure the time to reach dust-free (the time until a trace of the needle emerges from the inside to the surface of the coating film). The result is shown in Table 2. When the time is shorter, a curing rate is higher.

### <Resistance to water-containing methanol>

In the same manner as described above, an epoxy resin composition of each example was applied onto a base material (zinc phosphate-treated steel plate) using an applicator to form a coating film (film thickness just after application: 200 µm). This coating film was stored under conditions of 23°C and 50% R.H., and after a lapse of 7 days, a non-coating area was sealed with anticorrosive paints ("MILLION PRIMER" and "MILLION CLEAR" manufactured by Kansai Paint Co., Ltd.) to prepare a test specimen.

The test specimen was immersed in a 90% aqueous methanol solution under a condition of 23°C, and after a lapse of 1 week, the appearance of the test specimen was visually observed to evaluate resistance to water-containing methanol according to the following criteria. The result is shown in Table 2.
Ex: There is no change in appearance.
G: There is a slight reduction in gloss.
F: Surface roughness is observed, and there is a reduction in gloss.
P: Blister occurs or the coating film is dissolved.

### Example 1

### (Production of amino composition A)

### [Step (1)]

In a separable flask having an internal capacity of 300 milliliters and equipped with a stirrer, a thermometer, a nitrogen inlet tube, a dropping funnel, and a condenser, 40.0 g (0.3 mol) of m-xylylenediamine (MXDA manufactured by MITSUBISHI GAS CHEMICAL COMPANY, INC.) was charged, and 28.2 g (0.3 mol) of furfural (manufactured by Tokyo Chemical Industry Co., Ltd.) was added dropwise thereto for 15 minutes to 20 minutes in a nitrogen stream with stirring. After the completion of the dropwise addition, the temperature was raised to 80°C to perform a reaction for 30 minutes. The reaction was terminated after the disappearance of a ¹H peak derived from the aldehyde group of furfural was confirmed by ¹H-NMR measurement of a reaction product. Then, toluene was added to the reaction product, and moisture in the system was removed by azeotropic dehydration to obtain an imine as a reaction product obtained by reacting m-xylylenediamine and furfural in a molar ratio of 1/1.

### [Step (2)]

In an autoclave (capacity: 230 mL, material: SUS316L) equipped with a stirrer and a heater, 29.7 g of the imine obtained in the step (1), 30 g of toluene, and 0.3 g of a 5% Pd/C catalyst (manufactured by N.E. CHEMCAT CORPORATION; STD type) as a hydrogenation catalyst were charged, and a gas phase was replaced with hydrogen. Then, temperature rise was started while stirring was performed to achieve a liquid temperature of 80°C in 1 hour, and then the pressure was increased to 1 MPaG with hydrogen. Then, a reaction was continued for 1.5 hours under the condition of a liquid temperature of 80°C while hydrogen was supplied as needed in such a manner that the pressure was maintained at 1 MPaG. The thus obtained reaction liquid was filtered to remove the hydrogenation catalyst and then concentrated under vacuum to obtain 28.8 g of an amino composition a as a reduced product of the imine obtained in the step (1). The obtained amino composition a was subjected to distillation under reduced pressure to distill away remaining m-xylylenediamine to obtain 26.2 g of an amino composition A.

The obtained amino composition A was subjected to GC analysis and ¹H-NMR analysis under the above-described conditions to calculate its compositional ratio shown in Table 1. In the GC analysis, a solution obtained by dissolving 0.1 g of the amino composition A in 1 g of methanol was used as a measurement sample. It should be noted that a GC area ratio shown in Table 1 means the area percentage of a component detected by gas chromatography.

The content of an amino compound represented by the general formula (1) (amino compound (A)) in the amino composition A was 73% in terms of GC area ratio, the content of an amino compound represented by the general formula (3) (amino compound (B)) was 27% in terms of GC area ratio, and the molar ratio between the amino compound (A) and the amino compound (B) (A/B) was 84/16. The viscosity at 25°C and AHEW of the amino composition A are shown in Table 2.

The result of ¹H-NMR analysis of the amino composition A performed under the above-described conditions confirmed that, in the amino composition A, the amino compound represented by the general formula (1) (amino compound (A)) contained the compound represented by the following structural formula (A1) as a main component, and the amino compound represented by the general formula (3) (amino compound (B)) contained the compound represented by the following structural formula (B1) as a main component. It should be noted that the amino compound (A) contained a trace amount of a compound obtained by reducing a part or all of the unsaturated bonds of furan ring of the compound represented by the following structural formula (A1) in addition to the compound represented by the following structural formula (A1), and the amino compound (B) contained a trace amount of a compound obtained by reducing a part or all of the unsaturated bonds of furan rings of the compound represented by the following structural formula (B1) in addition to the compound represented by the following structural formula (B1).

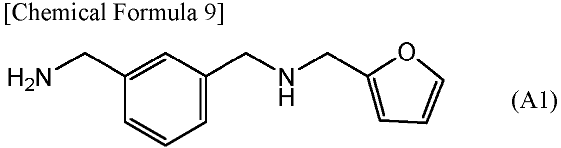

The ¹H-NMR chemical shift of the compound represented by the structural formula (A1): δ 7.36 ppm (dd, 1H, -O-CH=), δ 7.30-7.20 ppm (m, 4H, -C₆H₄-), δ 6.31 ppm (dd, 1H, - O-CH=CH-), δ 6.18 ppm (dd, 1H, -O-CH=CH-CH=), δ 3.85 ppm (s, 2H, -CH₂-NH₂), δ 3.78 ppm (d, 4H, -NH-CH₂-C₄H₃O, -C₆H₄-CH₂-NH-), δ 1.58 ppm (s, 3H, -NH-, -NH₂)

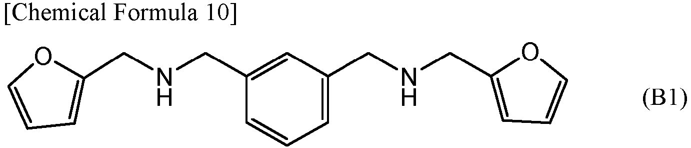

The ¹H-NMR chemical shift of the compound represented by the structural formula (B1): δ 7.36 ppm (dd, 2H, -O-CH=), δ 7.30-7.20 ppm (m, 4H, -C₆H₄-), δ 6.31 ppm (dd, 2H, - O-CH=CH-), δ 6.18 ppm (dd, 2H, -O-CH=CH-CH=), δ 3.78 ppm (d, 8H, -NH-CH₂-C₄H₃O, - C₆H₄-CH₂-NH-), δ 1.75 ppm (s, 2H, -NH-)

### (Preparation and evaluation of epoxy resin composition)

A liquid epoxy resin having glycidyloxy groups derived from bisphenol A ("jER828" manufactured by Mitsubishi Chemical Corporation, epoxy equivalent weight 186 g/eq.) was used as a main agent epoxy resin, and the above-described amino composition A was used as an epoxy resin curing agent.

The epoxy resin and the epoxy resin curing agent were blended according to a formulation shown in Table 2 and mixed with stirring at 23°C to prepare an epoxy resin composition. The ratio of the number of active hydrogen atoms in the epoxy resin curing agent to the number of epoxy groups in the main agent epoxy resin (the number of active hydrogen atoms in the epoxy resin curing agent/the number of epoxy groups in the main agent epoxy resin) was set to 1/1.

The obtained epoxy resin composition was used to make various evaluations according to the above-described methods. The results are shown in Table 2.

### Example 2

### (Production of amino composition B)

An amino composition B was produced in the same manner as in Example 1 except that in the step (1) in Example 1, the amount of m-xylylenediamine charged was changed to 40.9 g (0.3 mol) and the amount of furfural charged was changed to 43.2 g (0.45mol). The composition of the amino composition B is shown in Table 1, and the viscosity at 25°C and AHEW of the amino composition B are shown in Table 2.

### (Preparation and evaluation of epoxy resin composition)

An epoxy resin composition was prepared and evaluated in the same manner as in Example 1 except that the amino composition B was used instead of the amino composition A. The results are shown in Table 2.

### Example 3

### (Production of amino composition C)

An amino composition C was produced in the same manner as in Example 1 except that in the step (1) in Example 1, the amount of m-xylylenediamine charged was changed to 40.9 g (0.3 mol) and 33.0 g (0.3 mol) of 5-methylfurfural (manufactured by Tokyo Chemical Industry Co., Ltd.) was used instead of furfural. The composition of the amino composition C is shown in Table 1, and the viscosity at 25°C and AHEW of the amino composition C are shown in Table 2.

The result of ¹H-NMR analysis of the amino composition C performed under the above-described conditions confirmed that, in the amino composition C, the amino compound represented by the general formula (1) (amino compound (A)) was the compound represented by the following structural formula (A2), and an amino compound represented by the general formula (3) (amino compound (B)) was the compound represented by the following structural formula (B2).

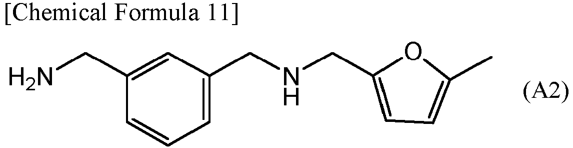

The ¹H-NMR chemical shift of the compound represented by the structural formula (A2): δ 7.30-7.19 ppm (m, 4H, -C₆H₄-), δ 6.04 ppm (dd, 1H, -O-C(CH₃)=CH-), δ 5.89-5.88 ppm (m, 1H, -O-C(CH₃)=CH-CH-), δ 3.86 ppm (s, 2H, -C₆H₄-CH₂-NH₂, ), δ 3.78 ppm (d, 2H, -C₆H₄-CH₂-NH-), δ 3.73 ppm, (d, 2H, -NH-CH₂-C₄H₂O-CH₃), δ 2.27 ppm (s, 3H, -CH₃), δ 1.55 ppm (s, 3H, -NH-, -NH₂)

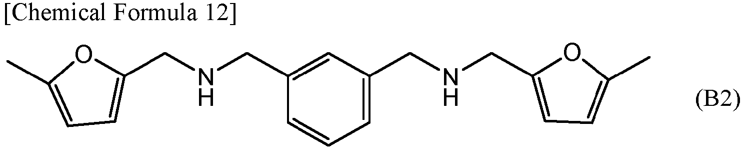

The ¹H-NMR chemical shift of the compound represented by the structural formula (B2): δ 7.30-7.19 ppm (m, 4H, -C₆H₄-), δ 6.04 ppm (dd, 2H, -O-C(CH₃)=CH-), δ 5.89-5.88 ppm (m, 2H, -O-C(CH₃)=CH-CH-), δ 3.78 ppm (d, 4H, -C₆H₄-CH₂-NH₂), δ 3.73 ppm, (d, 4H, -NH-CH₂-C₄H₂O-CH₃), δ 2.27 ppm (s, 6H, -CH₃), δ 1.67 ppm (s, 2H, -NH-, -NH₂)

### (Preparation and evaluation of epoxy resin composition)

An epoxy resin composition was prepared and evaluated in the same manner as in Example 1 except that the amino composition C was used instead of the amino composition A. The results are shown in Table 2.

### Comparative Example 1

### (Production of comparative amino composition D)

In a separable flask having an internal capacity of 300 milliliters and equipped with a stirrer, a thermometer, a nitrogen inlet tube, a dropping funnel, and a condenser, 40.8 g of m-xylylenediamine (MXDA manufactured by MITSUBISHI GAS CHEMICAL COMPANY, INC.) was charged, and a polyfunctional epoxy resin having glycidyloxy groups derived from bisphenol A ("jER828" manufactured by Mitsubishi Chemical Corporation, epoxy equivalent weight: 186 g/eq.) was added dropwise thereto as an epoxy compound in an amount of 22.3 g (corresponding to an amount such that the number of active hydrogen atoms in m-xylylenediamine /the number of epoxy groups in the epoxy resin compound = 10/1) for 1 hour in a nitrogen stream with stirring. After the completion of the dropwise addition, the temperature was raised to 80°C to perform a reaction for 2 hours to obtain a MXDA-jER828 reaction composition. Benzyl alcohol was added thereto for dilution in an amount corresponding to 40 mass% of the total mass. In this way, a comparative amino composition D whose concentration of the reaction composition was 60 mass% solution was obtained. The AHEW of the comparative amino composition D (as a whole containing benzyl alcohol) was 98. The viscosity at 25°C of the comparative amino composition D (as a whole containing benzyl alcohol) is shown in Table 2.

### (Preparation and evaluation of epoxy resin composition)

An epoxy resin composition was prepared and evaluated in the same manner as in Example 1 except that the comparative amino composition D was used instead of the amino composition A. The results are shown in Table 2.

### Comparative Example 2

As a comparative amino composition E, a reaction product of styrene and xylylenediamine ("Gaskamine 240" manufactured by MITSUBISHI GAS CHEMICAL COMPANY, INC., AHEW: 103) was used. The viscosity at 25°C of the comparative amino composition E is shown in Table 2.

An epoxy resin composition was prepared and evaluated in the same manner as in Example 1 except that the comparative amino composition E was used instead of the amino composition A. The results are shown in Table 2.

### Comparative Example 3

### (Production of comparative amino composition F)

A comparative amino composition F was produced in the same manner as in Example 1 except that in the step (1) in Example 1, the amount of m-xylylenediamine charged was changed to 27.2 g (0.2 mol), the amount of furfural charged was changed to 39.4 g (0.4 mol), and 66.3 g of toluene was added as a solvent. The composition of the comparative amino composition F is shown in Table 1, and the viscosity at 25°C and AHEW of the comparative amino composition F are shown in Table 2.

### (Preparation and evaluation of epoxy resin composition)

An epoxy resin composition was prepared and evaluated in the same manner as in Example 1 except that the comparative amino composition F was used instead of the amino composition A. The results are shown in Table 2.

### [Table 1]

**Table 1**

| | | | GC area ratio of amino compound (A) | GC area ratio of amino compound (B) | Molar ratio between amino compound (A) and amino compound (B) |
|---|---|---|---|---|---|
| | | | (%) | (%) | |
| Example | 1 | Amino composition A (MXDA/FAL-1) | 73 | 27 | 84/16 |
| | 2 | Amino composition B (MXDA/FAL-1.5) | 43 | 57 | 56/44 |
| | 3 | Amino composition C (MXDA/MFAL-1) | 78 | 22 | 80/20 |
| Comparative Example | 3 | Comparative amino composition F (MXDA/FAL-2) | 4 | 96 | 5/95 |

### [Table 2]

**Table 2**

| | | | | Example 1 | Example 2 | Example 3 | Comparative Example 1 | Comparative Example 2 | Comparative Example 3 |
|---|---|---|---|---|---|---|---|---|---|
| Epoxy resin composition | Curing agent | Amino composition A (MXDA/FAL-1) | | 31 | | | | | |
| | | Amino composition B (MXDA/FAL-1.5) | | | 36 | | | | |
| | | Amino composition C (MXDA/MFAL-1) | | | | 32 | | | |
| | | Comparative amino composition D (MXDAad51BA40) | (parts by mass) | | | | 35 | | |
| | | Comparative amino composition E (Gaskamine240) | | | | | | 36 | |
| | | Comparative amino composition F (MXDA/FAL-2) | | | | | | | 43 |
| | | Viscosity (25°C) | (mPa·s) | 55 | 73 | 59 | 370 | 66 | 110 |
| | | AHEW | (g/eq.) | 85 | 104 | 88 | 98 | 103 | 139 |
| | Main agent | jER828 | (parts by mass) | 69 | 64 | 68 | 65 | 64 | 57 |
| Evaluation results | Set to touch (1d/7d) | | - | Ex/Ex | Ex/Ex | Ex/Ex | Ex/Ex | Ex/Ex | Ex/Ex |
| | Pencil hardness (1d/7d) | | - | H/H | H/H | H/H | H/H | H/H | <6B/HB |
| | Water resistance spot (1d/7d) | | - | Ex/Ex | Ex/Ex | Ex/Ex | F/F | Ex/Ex | F/G |
| | Appearance (transparency/gloss) | | - | Ex/Ex | Ex/Ex | Ex/Ex | F/G | Ex/Ex | Ex/Ex |
| | RCI drying time (dust free) | | (hours: minutes) | 6:30 | 10:00 | 6:00 | 2:30 | 7:30 | 23:30 |
| | Resistance to water-containing methanol | | - | Ex | Ex | G | P | F | P |

Table 2 confirms that each of the epoxy resin curing agents containing the amino composition of the present invention has low viscosity, and the coating film of each of the epoxy resin compositions using such an epoxy resin curing agent has excellent resistance to water-containing methanol.

### Industrial Applicability

The present invention makes it possible to provide an amino composition that has low viscosity and can be used as an epoxy resin curing agent to form a coating film having excellent resistance to water-containing methanol and a method for producing the same, an epoxy resin curing agent containing the composition, an epoxy resin composition, and a cured product thereof.

## Claims

1. An amino composition comprising an amino compound (A) represented by the following general formula (1) and an amino compound (B) represented by the following general formula (3), wherein a molar ratio between the amino compound (A) and the amino compound (B) [(A)/(B)] in the composition is 10/90 to 99/1,
NH₂-CH₂-X-CH₂-NH-R¹ (1)
wherein R¹ is a monovalent group represented by the following general formula (2) and X is a phenylene group, 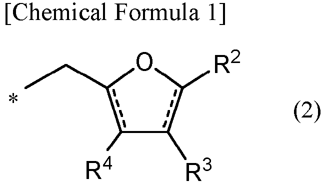
wherein a dashed line indicates presence or absence of a π bond, R² to R⁴ are each independently a hydrogen atom or an alkyl group that has 1 to 4 carbon atoms and optionally has a hydroxy group, and * represents a bonding position,
R¹-NH-CH₂-X-CH₂-NH-R¹ (3)
wherein R¹ and X are the same as above.

2. An epoxy resin curing agent comprising the amino composition according to claim 1.

3. An epoxy resin composition comprising an epoxy resin and the epoxy resin curing agent according to claim 2.

4. A cured product of the epoxy resin composition according to claim 3.

5. A method for producing the amino composition according to claim 1, the method comprising the following step (1) and step (2) in this order:
step (1): the step of reacting a diamine represented by the following general formula (4) and an aldehyde compound represented by the following general formula (5) to obtain an imine; and
step (2): the step of reducing the imine obtained in the step (1),
NH₂-CH₂-X-CH₂-NH₂ (4)
wherein X is the same as above, 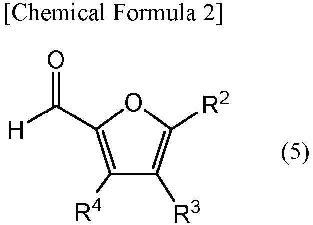
wherein R² to R⁴ are the same as above.

6. The method for producing the amino composition according to claim 5, wherein in the step (1), 0.8 to 1.8 mol of the aldehyde compound represented by the above general formula (5) is reacted per mol of the diamine represented by the above general formula (4).
